# EUROPEAN PATENT APPLICATION

(11) **EP 4 529 917 A1**
(43) Date of publication of application: **02.04.2025**
(21) Application number: 24165526.5
(22) Date of filing: 22.03.2024
(51) Int. Cl.: A61K 9/00

(54) **TOPICAL DELIVERY COMPOSITIONS COMPRISING NON-STEROIDAL ANTI-INLAMMATORY DRUGS**

(30) Priority: 27.09.2023 US 202318373612
(71) Applicant: Andros Pharmaceuticals Co., Ltd., Zhubei City, Hsinchu County 302058 (TW)
(72) Inventor: HU, Chun Wen, 302058 Zhubei City, Hsinchu County (TW); WANG, Ae June, 302058 Zhubei City, Hsinchu County (TW); YEN, Mei Wen, 302058 Zhubei City, Hsinchu County (TW)
(74) Representative: Becker, Eberhard

(57) **Abstract**

The present disclosure relates generally to topical delivery compositions for improved topical composition comprising at least one active agent, at least one phospholipid and urea. The active agent of the present disclosure comprises non-steroid anti-inflammatory drugs (NSAIDs). The topical delivery compositions of the present disclosure improve the solubility of the NSAIDs. The present disclosure also relates to a method for preparation of a topical composition of the present disclosure. The topical compositions are useful in treating inflammation, arthritis and/or pain, or conditions for which the signs and symptoms include inflammation, arthritis and/or pain, by topical administration to a subject.

## Description

### TECHNICAL FIELD

The present disclosure is related generally to an improved topical delivery composition that can be used in the treatment of inflammation, arthritis and/or pain, or conditions for which the signs and symptoms include inflammation, arthritis and/or pain.

### BACKGROUND

Oral non-steroidal anti-inflammatory drugs (NSAIDs) have analgesic, anti-inflammatory and antipyretic effects and are useful in reducing pain and inflammation. NSAIDs are however associated with serious potential side effects including nausea, vomiting, peptic ulcer disease, GI hemorrhage, and cardiovascular events.

When NSAIDs are applied topically, local drug concentration in muscle and joint tissues is significantly higher than in non-treated sites. Additionally, there is no intensive metabolism in liver (so called first pass effect) because such drugs do not pass through the liver before action. The required amount of NSAIDs is lower than an oral dose to achieve similar anti-inflammatory and analgesic effects.

Diclofenac is a well-known and widely used NSAID, it initially developed as sodium salt for oral or topically applications as cream or plasters. Diclofenac sodium can be easily formulated as powder for oral formulation, but due to its low water solubility the formulations in gel or plasters can be problematic. Moreover, the effectiveness of topical administration of diclofenac is limited by the difficulty of this drug to permeate the skin.

Voltaren gel is a 1% diclofenac sodium emulgel, it was approved by the Food and Drug Administration (FDA) in 2007. Diclofenac sodium dissolved in lipid phase and mixed with nonionic surfactant to form an emulsion (US 7732489). However, in order to increase diclofenac concentration for decrease applied amount, it needs additional C2-C4 alkanol and glycol solvent (WO 2016038556103).

US 4575515 and US 4652557 disclose topical NSAID compositions, one of which, consisting of 1.5% diclofenac sodium, 45.5% dimethylsulphoxide (DMSO), 11.79% ethanol, 11.2% propylene glycol, 11.2% glycerine, and water. This formulation used lots of solvent, especially DMSO, for improving solubility and skin permeability.

EP 1003499 discloses a pharmaceutical preparation for topical application containing diclofenac as an active substance which is dissolved in a solvent mixture, containing at least one alkyl alcohol with 2 to 4 C atoms as a main constituent, at least one short-chain N alkyl pyrrolidone and at least one pyrrolidone substituted with a long-chain alkyl radical.

WO 2010/060798 discloses a pharmaceutical formulation which comprises an aqueous solution comprising from 1% to 5% (w/v) of a pharmaceutically acceptable salt of diclofenac, from 3% to 30% (w/v) of at least one polyoxyalkylene ester of hydroxyl fatty acid, water as the main component, and optionally, a co-solvent.

US 7138394 discloses a eutectic mixture of camphor, menthol, thymol and similar compounds is a powerful solvent for NSAIDs and other substances. The solubility of Indomethacin, Diclofenac, or Ketoprofen in the mixture increased between 3 and 20-fold.

WO 2014/009793 discloses a volatile-silicone-based pharmaceutical or veterinary composition containing, as an active ingredient, diclofenac or a diclofenac organic base salt which, when dissolved in said volatile silicone base, exerts its anti-inflammatory and analgesic activity upon a topical application involving a deep penetration of the active ingredient through the skin.

EP 834312 discloses a medicament based on diclofenac or its salts, for topical treatment of inflammation and pain, containing at least one solvent and at least one solubilizer. The solvent is a mixture of water, diethyleneglycol monoethyl ether and optionally C2-6 polyalcohols and polyesters thereof, the esters and ethers thereof, as well as glycerides and/or ethoxylated derivatives thereof. The solubilizer is at least one phospholipid.

US 5958379 discloses a sprayable liquid pharmaceutical composition containing at least one active substance, diclofenac sodium, and comprises soybean lecithin as solubilizer and gel-forming agent. Alcohol and glycols comprise as co-solvents for easily vaporizable.

US 5738869 specifically describes a phospholipid transdermal drug delivery system containing α-tocopherol, aliphatic alcohol and diclofenac.

The improved topical drug delivery system is well established, and one recognized approach is the use of penetration enhancers. These agents decrease the skin's barrier resistance and thus allow for improved penetration of active medication. Diclofenac sodium topical solution 1.5% or 2% w/w (PENNSAID) is indicated for the treatment of signs and symptoms of osteoarthritis of the knee(s) (hereinafter referred as knee OA). Additional absorption enhancing ingredients in this product include 45.5% DMSO U.S. Pharmacopeia Convention, propylene glycol, and alcohol. Topical diclofenac solution with DMSO is indicated for the treatment of the signs and symptoms of knee OA. Its clinical efficacy and safety have been demonstrated in multiple clinical trials for treatment of the signs and symptoms of knee OA, including demonstrated improvement in pain and impaired ability to perform daily activities and overall general health. In addition, two randomized, controlled trials have demonstrated that this formulation of topical diclofenac provides equivalent efficacy with fewer GI adverse events and changes in renal and hepatic laboratory values compared with therapeutic doses of oral diclofenac.

However, skin-related events were the most common treatment-emergent adverse events in patients who received PENNSAID in vehicle- and placebo- controlled clinical trials. Dry skin, which was the most common adverse event, occurred in 18.2-39.3% of PENNSAID recipients and was significantly (p < 0.05) more common in this treatment group than in topical placebo and vehicle control recipients. Other common skin-related adverse events reported in clinical trials included rash, paranesthesia and pruritus. In the equivalence study, skin-related adverse events (dry skin, rash, pruritus and vesiculobullous rash) were experienced by significantly more PENNSAID than oral diclofenac recipients. Using large amount solvents could increase the diclofenac solubility and bioavailability, and could ether increase the risk of skin irritation and sensitization.

Thus, there is a need in the art for a novel topical delivery composition to reduce the risk of skin irritation and sensitization.

### SUMMARY

The object of the present disclosure is to provide a novel topical delivery composition in which the solubility of the non-steroid anti-inflammatory drug (NSAID) is improved and remains stable throughout the shelf-life of the composition. Another object of the present disclosure is to develop a novel topical delivery composition that can promote the penetration of the non-steroid anti-inflammatory drug (NSAID) through the skin and the skin irritation is also reduced.

The present disclosure relates to topical delivery compositions for treating inflammation, arthritis and/or pain, or for treating conditions for which the signs and symptoms include inflammation, arthritis and/or pain. In an embodiment, the present disclosure provides a topical composition comprising at least one active agent, at least one phospholipid and urea, wherein the active agent comprises a non-steroid anti-inflammatory drug (NSAID).

In an embodiment, the active agent is present at an amount of between about 0.1 wt% to about 10.0 wt% of the total composition.

In some embodiments, the non-steroid anti-inflammatory drug (NSAID) of the present disclosure is selected from the group consisting of acetylsalicylicacid, sodium salicylate, choline magnesium trisalicylate, salsalate, diflunisal, salicylsalicylic acid, sulfasalazine, olsalazin, acetaminophen, indomethacin, sulindac, etodolac, tolmetin, diclofenac, ketorolac, ibuprofen, naproxen, flurbiprofen, ketoprofen, aceclofenac, fenoprofen, oxaprozin, mefenamic, meclofenamic acid, piroxicam, meloxicam, tenoxicam, phenylbutazone, oxyphenbutazone, nabumetone, rofecoxib, celecoxib, any pharmaceutically acceptable salt, derivative and mixtures thereof.

In an embodiment, the phospholipid is present at an amount of between about 0.1 wt% to about 20.0 wt% of the total composition.

In an embodiment, the surfactant of the present disclosure is phospholipid. In some embodiments, the phospholipid is selected from the group consisting of phosphatidyl choline (PC), dipalmitoylphosphatidylcholine (DPPC), distearoyl phosphatidyl choline (DSPC), palmytoyl stearoyl phosphatidyl choline (PSPC), palmitoyl oleyl phosphatidyl choline (POPC), dioleyl phosphatidylcholine (DOPC), stearoyl oleyl phosphatidylcholine (SOPC), cardiolipin, plasmalogen, lyso phosphatidyl choline (LPC), phosphatidyl ethanolamine (PE) (Cephalin), phosphatidyl inositol (PI), phosphatidyl serine (PS), lecithin, lysolecithin, soy lecithin, rapeseed lecithin, corn or sunflower lecithin, egg lecithin, Epicorn 200, Epicorn 100, phospholipone 90G, LIPOID R-100 (Rapeseed), LIPOID H-100 (Sunflower), LIPOID-S100 (Soybean), LIPOID-S75, Phosal 50PG, and combinations thereof.

In an embodiment, the urea is present at an amount of between about 5.0 wt% to about 50.0 wt% of the total composition.

In an embodiment, the topical composition of the present disclosure further comprises a lower chain alcohol having carbon chain length of C2 to C5. In some embodiments, the lower chain alcohol is selected from the group consisting of ethanol, propanol, isopropanol, butanol, isobutanol, pentanol, isopentanol, ethylene glycol, propylene glycol, butylene glycol, isobutylene glycol, pentylene glycol, isopentylene glycol and combinations thereof.

In an embodiment, the topical composition of the present disclosure further comprises a pH adjusting agent. In some embodiments, the pH adjusting agent is selected from the group consisting of sodium phosphate, disodium phosphate, sodium bicarbonate, tris(hydroxymethyl)aminomethane, boric acid, sodium hydroxide, hydrochloride, triethylamine , citric acid, alanine, glycine, leucine, lactic acid, phenol and combinations thereof.

In some embodiments, the composition of the present disclosure is in the form of cream, ointment, gel, transdermal formulations, foam, spray, lotion, solution, emulsion or suspension.

In an embodiment, the present disclosure provides a topical composition comprising about 0.1 wt% to about 10.0 wt% of active agent, about 0.1 wt% to about 20.0 wt% of phospholipid and about 5.0 wt% to about 50.0 wt% of urea, wherein the active agent comprises a non-steroid anti-inflammatory drug (NSAID).

In an aspect, a method for preparation of a topical composition of the present disclosure is provided comprising the steps of (a) dissolving at least one of active agent and at least one of phospholipid to form an oil phase, (b) adding urea to water to form an aqueous phase, and (c) mixing the oil phase and the aqueous phase to form a homogenous composition.

In another aspect, a method for treating inflammation, arthritis and/or pain, or conditions for which the signs and symptoms include inflammation, arthritis and/or pain, comprising topically administering to a subject in need thereof a topical composition of the present disclosure, wherein the composition of the present disclosure comprises at least one of active agent, at least one of phospholipid and urea.

Additional aspects and advantages will appear from the drawings and the detailed description.

### BRIEF DESCRIPTION OF THE DRAWINGS

The present disclosure will be better understood from the following detailed description read in light of the accompanying drawings, where:
FIG. 1 is a graphical comparation of the skin permeability of Formulation 9, Formulation 23 and Formulation 24.
FIG. 2 is a graphical comparation of the skin permeability of Formulation 25-80 ng/BID and Formulation 25-160 ng/QD.
FIG. 3 is a graphical comparation of the skin permeability of 4% diclofenac solution (Formulation 30) and 2% Pennsaid.

### DESCRIPTION OF EMBODIMENTS

The following disclosure provides many different embodiments, or examples, for implementing different features of the provided subject matter. Specific examples are described below to simplify the present disclosure. These are, of course, merely examples and are not intended to be limited.

Unless defined otherwise, all technical and scientific terms used herein have the same meaning as commonly understood to one of ordinary skill in the art to which the present disclosure pertains. Otherwise, certain terms used herein have the meanings as set forth in the specification.

The singular forms "a," "an," and "the" are used herein to include plural referents unless the context clearly dictates otherwise.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in the respective testing measurements. Also, as used in, the term "about" generally means within 10.0%, 5.0%, 1.0%, or 0.5% of a given value or range. Thus, a numerical value typically includes ± 10% of the recited value. For example, a concentration of 1 mg/mL includes 0.9 mg/mL to 1.1 mg/mL. Likewise, a concentration range of 1.0 wt% to 10.0 wt% includes 0.9 wt% to 11.0 wt%. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperature, operating conditions, ratios of amounts, and the like thereof disclosed herein should be understood as modified in all instances by the term "about". Accordingly, unless indicated to the contrary, the numerical parameters set forth in the present disclosure and attached claims are approximations that can vary as desired. At the very last, each numerical parameter should at least be construed in light of the number of reported significant digits and by applying ordinary rounding techniques. Ranges can be expressed herein as from one endpoint to another endpoint or between two endpoints. All ranges disclosed herein are inclusive of the endpoints, unless specified otherwise.

### 1. DEFINITIONS

For convenience, certain terms employed in the context of the present disclosure are collected herein. Unless defined otherwise, all technical and scientific terms used herein have the same meaning as commonly understood by one of the ordinary skills in the art to which the present disclosure belongs.

The term "treatment" as used herein are intended to mean obtaining a desired pharmacological and/or physiologic effect, e.g., blocking the action of cyclo-oxygenase enzyme. The effect may be prophylactic in terms of completely or partially preventing a disease or symptom thereof and/or therapeutic in terms of a partial or complete cure for a disease and/or adverse effect attributable to the disease. "Treatment" as used herein includes preventive (e.g., prophylactic), curative or palliative treatment of a disease in a mammal, particularly human; and includes: (1) preventative (e.g., prophylactic), curative or palliative treatment of a disease or condition (e.g., arthritis or osteoarthritis) from occurring in an individual who may be pre-disposed to the disease but has not yet been diagnosed as having it; (2) inhibiting a disease (e.g., by arresting its development); or (3) relieving a disease (e.g., reducing symptoms associated with the disease).

The term "administered", "administering" or "administration" are used interchangeably herein to refer a mode of delivery, including, without limitation, intravenously, intramuscularly, intraperitoneally, intraarterially, intracranially, or subcutaneously administering an agent (e.g., the present glycoconjugate vaccine) that confers immunoprotecting to a subject from a bacterial infection.

The term "an effective amount" as used herein refers to an amount effective, at dosages, and for periods of time necessary, to confer a therapeutic effect on the treated subject. An effective amount of an agent is not required to cure a disease or condition but will provide a treatment for a disease or condition such that the onset of the disease or condition is delayed, hindered or prevented, or the disease or condition symptoms are ameliorated. The specific effective or sufficient amount will vary with such factors as the particular condition being treated, the physical condition of the patient (e.g., the patient's body mass, age, or gender), the type of mammal or animal being treated, the duration of the treatment, the nature of concurrent therapy (if any), and the specific formulations employed and the like. Effective amount may be expressed, for example, as the total mass of the active agent (e.g., in grams, milligrams or micrograms) or a ratio of mass of the active agent to body mass, e.g., as milligrams per kilogram (mg/kg). The effective amount may be divided into one, two or more doses in a suitable form to be administered at one, two or more times throughout a designated time period.

The term "pharmaceutically acceptable" refers to molecular entities and compositions that are "generally regarded as safe", e.g., that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "subject" or "patient" is used interchangeably herein and is intended to mean a mammal including the human species that is treatable by the compound of the present disclosure. The term "mammal" refers to all members of the class Mammalia, including humans, primates, domestic and farm animals, such as rabbit, pig, sheep, and cattle; as well as zoo, sports or pet animals; and rodents, such as mouse and rat. Further, the term "subject" or "patient" intended to refer to both the male and female gender unless one gender is specifically indicated. Accordingly, the term "subject" or "patient" comprises any mammal which may benefit from the treatment method of the present disclosure. Examples of a "subject" or "patient" include, but are not limited to, a human, rat, mouse, guinea pig, monkey, pig, goat, cow, horse, dog, cat, bird and fowl. In a preferred embodiment, the subject is a human.

The terms "comprise", "comprises", and "comprising" are used interchangeably herein to be interpreted inclusively rather than exclusively. The terms "consist", "consisting", "consisting essentially of" and its variants, are to be interpreted exclusively, rather than inclusively.

As used herein, unless stated otherwise, the term "ambient conditions" refers to atmospheric pressure and a temperature of 22-24°C .

As used herein, a "composition" or "formulation" of the present disclosure refers to a mixture of excipients or other chemicals prepared according to a specific recipe and preparation procedure. A formulation constitutes the final or intermediate form of a drug, cosmetic or supplement product, such as solution, gel, or foam etc. A composition may optionally contain a drug. The terms of "composition" and "formulation" are used interchangeably in this application.

### 2. DETAIL DESCRIPTION OF EMBODIMENTS

The present disclosure provides one or more topical compositions comprising at least one of active agent, at least one phospholipid and urea; methods of manufacturing of the topical compositions, methods of treating, and dosage forms. The active agent is configured to inhibit cyclo-oxygenase enzyme activity such that the pharmaceutical compositions are capable of treating conditions associated with cyclo-oxygenase enzyme activity, including, for example, inflammation, arthritis and/or pain when administered topically.

In one embodiment, the present disclosure provides a topical composition comprising at least one active agent, at least one phospholipid and urea, wherein the active agent comprises a non-steroid anti-inflammatory drug (NSAID).

In certain embodiments, the non-steroid anti-inflammatory drug (NSAID) of the present disclosure is selected from the group consisting of acetylsalicylicacid, sodium salicylate, choline magnesium trisalicylate, salsalate, diflunisal, salicylsalicylic acid, sulfasalazine, olsalazin, acetaminophen, indomethacin, sulindac, etodolac, tolmetin, diclofenac, ketorolac, ibuprofen, naproxen, flurbiprofen, ketoprofen, aceclofenac, fenoprofen, oxaprozin, mefenamic, meclofenamic acid, piroxicam, meloxicam, tenoxicam, phenylbutazone, oxyphenbutazone, nabumetone, rofecoxib, celecoxib, any pharmaceutically acceptable salt, derivative and mixtures thereof.

In certain embodiments, the active agent of the present disclosure comprises diclofenac or pharmaceutically acceptable salts thereof. In one embodiment, the pharmaceutically acceptable salt is the diclofenac sodium salt. In certain embodiments, the pharmaceutically acceptable salt is the methanesulfonate salt.

In certain embodiments, the active agent of the present disclosure is present at an amount of between about 0.1 wt% to about 10.0 wt% of the total composition. In certain embodiments, the active agent of the present disclosure is present at an amount of between about 2.0 wt% to about 10.0 wt% of the total composition; for example, about 2.0wt%, about 3.0 wt%, about 4.0 wt%, about 5.0 wt%, about 6.0 wt%, about 7.0 wt%, about 8.0 wt%, about 9.0 wt% or about 10.0 wt% of the total composition. In certain embodiments, the active agent is present at an amount of between 0.5 wt% to about 10.0 wt% of the total composition; for example about 0.5 wt%, about 1.0 wt%, about 1.5 wt%, about 2.0 wt%, about 2.5 wt%, about 3.0 wt%, about 3.5 wt%, about 4.0 wt%, about 4.5 wt%, about 5.0 wt%, about 5.5 wt%, about 6.0 wt%, about 6.5 wt%, about 7.0 wt%, about 7.5 wt%, about 8.0 wt%, about 8.5 wt%, about 9.0 wt%, about 9.5 wt%, about 10.0 wt% of the total composition.

In certain embodiments, the composition of the present disclosure comprises phospholipid. Phospholipid refers to trimester of glycerol with two fatty acid and one phosphate ion. It is also known that phospholipids can increase the moisture content of the skin and the permeation of drugs through the skin. In some embodiments, the phospholipid may be a glycerophospholipid being selected from mono-phosphatidyl glycerols, bis-phosphatidyl glycerols, and tris-phosphatidyl glycerols. In certain embodiments, the phospholipid of the present disclosure is selected from the group consisting of phosphatidyl choline (PC), dipalmitoylphosphatidylcholine (DPPC), distearoyl phosphatidyl choline (DSPC), palmytoyl stearoyl phosphatidyl choline (PSPC), palmitoyl oleyl phosphatidyl choline (POPC), dioleyl phosphatidylcholine (DOPC), stearoyl oleyl phosphatidylcholine (SOPC), cardiolipin, plasmalogen, lyso phosphatidyl choline (LPC), phosphatidyl ethanolamine (PE) (Cephalin), phosphatidyl inositol (PI), phosphatidyl serine (PS), lecithin, lysolecithin, soy lecithin, rapeseed lecithin, corn or sunflower lecithins, egg lecithin, Epicorn 200, Epicorn 100, phospholipone 90G, LIPOID R-100 (Rapeseed), LIPOID H-100 (Sunflower), LIPOID-S100 (Soybean), LIPOID-S75, Phosal 50PG, and combinations thereof.

In certain embodiments, the phospholipid of the present disclosure is present at an amount of between about 0.1 wt% to about 20.0 wt% of the total composition. In certain embodiments, the phospholipid of the present disclosure is present at an amount of between about 1.0 wt% to about 10.0 wt% of the total composition, for example, about 1.0 wt%, about 1.5 wt%, about 2.0 wt%, about 2.5 wt%, about 3.0 wt%, about 3.5 wt%, about 4.0 wt%, about 4.5 wt%, about 5.0 wt%, about 5.5 wt%, about 6.0 wt%, about 6.5 wt%, about 7.0 wt%, about 7.5 wt%, about 8.0 wt%, about 8.5 wt%, about 9.0 wt%, about 9.5 wt% or about 10.0 wt% of the total composition. In certain embodiments, the phospholipid of the present disclosure is present at an amount of between about 0.1 wt% to about 15.0 wt% of the total composition. In certain embodiments, the phospholipid of the present disclosure is present at an amount of between about 0.1 wt% to about 10.0 wt% of the total composition. In certain embodiments, the phospholipid of the present disclosure is present at an amount of between about 0.1 wt% to about 5.0 wt% of the total composition.

It is well known that urea possesses skin exfoliating properties, which are useful in the control of passage of the active of the present disclosure through the dermal barrier. In certain embodiments, the urea is present at an amount of between about 5.0 wt% to about 50.0 wt% of the total composition. In certain embodiments, the urea is present at an amount of between about 5.0 wt% to about 35.0 wt% of the total composition; for example, about 5.0 wt%, about 10.0 wt%, about 15.5 wt%, about 20.0 wt%, about 25.0 wt%, about 30.0 wt%, or about 35.5 wt%. In certain embodiments, the urea is present at an amount of between about 10.0 wt% to about 30.0 wt%; for example, about 10.0 wt%, about 15.5 wt%, about 20.0 wt%, about 25.0 wt%, about 30.0 wt%, or about 35. wt% of the total composition. In certain embodiments, the urea is present at an amount of between about 5.0 wt% to about 45.0 wt% of the total composition. In certain embodiments, the urea is present at an amount of between about 5.0 wt% to about 40.0 wt% of the total composition. In certain embodiments, the urea is present at an amount of between about 5.0 wt% to about 35.0 wt% of the total composition. In certain embodiments, the urea is present at an amount of between about 5.0 wt% to about 30.0 wt% of the total composition. In certain embodiments, the urea is present at an amount of between about 5.0 wt% to about 25.0 wt% of the total composition. In certain embodiments, the urea is present at an amount of between about 5.0 wt% to about 20.0 wt% of the total composition.

In order to dispersing the phospholipid in the aqueous phase well, in certain embodiments, the topical composition of the present disclosure further comprises a lower chain alcohol having carbon chain length of C2 to C5. In certain embodiments, the lower chain alcohol of the present disclosure is selected from the group consisting of ethanol, propanol, isopropanol, butanol, isobutanol, pentanol, isopentanol, ethylene glycol, propylene glycol, butylene glycol, isobutylene glycol, pentylene glycol, isopentylene glycol and combinations thereof. In one embodiment, the lower chain alcohol of the present disclosure is ethanol.

The lower chain alcohol also serves as an effective penetration enhancer to enhance the bioavailability of diclofenac. However, excessive alcohol increases the risk of skin irritation. In certain embodiments, a lower chain alcohol of the present disclosure is present at an amount of between about 5.0 wt% to about 30.0 wt% of the total composition; for example about 5.0 wt%, about 10.0 wt%, about 15.0 wt%, about 20.0 wt%, about 25.0 wt% or about 30.0 wt% of the total composition. In certain embodiments, a lower chain alcohol of the present disclosure is present at an amount of between about 5.0 wt% to about 20.0 wt% of the total composition; for example about 5.0 wt%, about 10.0 wt%, about 15.0 wt%, or about 20.0 wt% of the total composition. In one embodiment, a lower chain alcohol of the present disclosure is present at an amount of about 10 wt% of the total composition.

In certain embodiments, the topical composition of the present disclosure further comprises a pH adjusting agent. Diclofenac is unstable in acidic environment, and it accelerates the generation of diclofenac impurities. The ideal pH shall be control in the range of about pH 7 to pH 9, and addition of the pH adjusting agent is necessary. In certain embodiments, the pH adjusting agent of the present disclosure is selected from the group consisting of sodium phosphate, disodium phosphate, sodium bicarbonate, tris(hydroxymethyl)aminomethane, boric acid, sodium hydroxide, hydrochloride, triethylamine, citric acid, alanine, glycine, leucine, lactic acid, phenol and combinations thereof. In one embodiment, the pH adjusting agent of the present disclosure comprises boric acid.

In certain embodiments, the pH adjusting agent of the present disclosure is present at an amount of between about 0.5 wt% to about 1.5 wt%; for example about 0.5 wt%, about 0.6 wt%, about 0.7 wt%, about 0.8 wt%, about 0.9 wt%, about 1.0 wt%, about 1.1 wt%, about 1.2 wt%, about 1.3 wt%, about 1.4 wt%, or 1.5 wt% of the total composition. In certain embodiments, the pH adjusting agent of the present disclosure is present at an amount of between about 0.5 wt% to about 1.0 wt% of the total composition; for example about 0.5 wt%, about 0.6 wt%, about 0.7 wt%, about 0.8 wt%, about 0.9 wt%, or about 1.0 wt% of the total composition. In one embodiment, the pH adjusting agent of the present disclosure is present at an amount of about 1.0 wt% of the total composition.

In certain embodiments, the topical composition of the present disclosure further comprises a thickener. In certain embodiments, the thickener is selected from the group consisting of Carbopol, xanthan gum, polyvinyl alcohol (PVA), polyvinylpyrrolidone (PVP), methyl cellulose (MC), ethyl cellulose (EC), hydroxypropyl methylcellulose (HPMC), hydroxypropyl cellulose (HPC), carboxymethyl cellulose (CMC), chitosan, alginate, hyaluronic acid, and combination thereof.

In certain embodiments, the topical composition of the present disclosure further comprises additives such as chelating agents, antioxidants, and preservatives. In certain embodiments, the chelating agent is selected from the group consisting of ethylenediaminetetraacetic acid (EDTA), EDTA derivatives, and combination thereof. In certain embodiments, the antioxidant is selected from the group consisting of ascorbic acid, stearic acid esters, sodium ascorbate, tocopherol (d-, 1- and dl-isomers of α-tocopherol, β-tocopherol, γ-tocopherol, δ-tocopherol, etc.) and ester derivatives thereof, nordihydroguaiaretic acid, butylhydroxytoluene, butylhydroxyanisole, tert-b utylhydroquinone and 1-oxo-3-methyl-4-isopropylbenzene, and combinations thereof. In certain embodiments, the preservative is selected from the group consisting of benzoic acid, benzyl alcohol, sodium benzoate, ethyl p-hydroxybenzoate, propyl p-hydroxybenzoate, butyl p-hydroxybenzoate, isothiazolinone, parabens such as methylparaben and propylparaben, and combinations thereof. In certain embodiments, the preservative is selected from the group consisting of tocopherol, tocopherol acetate, ascorbic acid, ascorbyl palmitate, propyl gallate, butylated hydroxytoluene (BHT), butylated hydroxylanisole (BHA), tertiary butylhydroquinone (TBHQ), ethylenediaminetetraacetic acid (EDTA). methylparaben (MP), ethylparaben (EP), propylparaben (PP), butylparaben (BP), benzoic acid, benzyl alcohol, and combination thereof.

In certain embodiments, the topical composition of the present disclosure is in the form of cream, ointment, gel, transdermal formulations, foam, spray, lotion, solution, emulsion or suspension.

In certain embodiments, the present disclosure provides a topical composition comprising 0.1 wt% to 10.0 wt% of active agent, 0.1 wt% to 20.0 wt% of phospholipid and 5.0 wt% to 50.0 wt% of urea, wherein the active agent comprises a non-steroid anti-inflammatory drug (NSAID).

In certain embodiments, the present disclosure provides a topical composition comprising 2.0 wt% to 10.0 wt% of active agent, 1.0 wt% to 10.0 wt% of phospholipid and 5.0 wt% to 35.0 wt% of urea, wherein the active agent comprises a non-steroid anti-inflammatory drug (NSAID).

In another embodiment, the present disclosure also provides a method for preparation of a topical composition of the present disclosure, comprising (a) dissolving at least one active agent and at least one phospholipid to form an oil phase, (b) adding urea to water to form an aqueous phase, and (c) mixing the oil phase and the aqueous phase to form a homogenous composition, wherein the active agent comprises a non-steroid anti-inflammatory drug (NSAID).

In still a further embodiment, the present disclosure further provides a method for treating inflammation, arthritis and/or pain, or conditions for which the signs and symptoms include inflammation, arthritis and/or pain, comprising: topically administering to a subject in need thereof a topical composition of the present disclosure, wherein the composition comprising at least one active agent, at least one phospholipid and urea and the active agent comprises a non-steroid anti-inflammatory drug (NSAID).

In an embodiment, the topical composition of the present disclosure may be utilized for treating inflammation, arthritis and/or pain, and for treating conditions for which the signs and symptoms include inflammation, arthritis and/or pain. In certain embodiments, the arthritis and/or pain, may include but is not limited to arthritis associated with joint pain and stiffness. The most common types of arthritis are osteoarthritis and rheumatoid arthritis. Though not wishing to be bound by the following theory, it is believed that administration of the topical composition of the present disclosure minimizes pain and/or motor impairment and/or administration of the topical composition of the present disclosure inhibits cyclo-oxygenase enzyme activity. In an embodiment, the dosing regimen for treating inflammation, the dosing regimen for treating inflammation, arthritis and/or pain comprises application to the affected area of the skin twice daily.

In another embodiment, the present disclosure provides a topical composition of the present disclosure for use in treating inflammation, arthritis and/or pain, or conditions for which the signs and symptoms include inflammation, arthritis and/or pain, wherein the topical composition comprises at least one active agent, at least one phospholipid and urea and the active agent comprises a non-steroid anti-inflammatory drug (NSAID).

In still another embodiment, the present disclosure also provides use of a topical composition of the present disclosure for preparation of a medicament for use in treating inflammation, arthritis and/or pain, or conditions for which the signs and symptoms include inflammation, arthritis and/or pain, wherein the topical composition comprises at least one active agent, at least one phospholipid and urea and the active agent comprises a non-steroid anti-inflammatory drug (NSAID).

In yet another embodiment, the present disclosure also provides a topical composition of the present disclosure for the treatment of inflammation, arthritis and/or pain, or conditions for which the signs and symptoms include inflammation, arthritis and/or pain, wherein the topical composition comprises at least one active agent, at least one phospholipid and urea and the active agent comprises a non-steroid anti-inflammatory drug (NSAID).

In certain embodiments, the topical composition of the present disclosure are stable upon storage in a closed container at: about 30 degrees Celsius and about 90 percent relative humidity for a period of at least about 20 hours; about 40 degrees Celsius and about 60 percent relative humidity for a period of at least about one week, two weeks or three weeks; about 25 degrees Celsius and about 60 percent relative humidity for a period of at least about one month; about 40 degrees Celsius and about 75 percent relative humidity for a period of at least 3 months; about 25 degrees Celsius and about 75 percent relative humidity for a period of at least about 3 months; or 15 degrees Celsius at any relative humidity for a period of at least about 3 months.

The foregoing outlines features of several embodiments so that those skilled in the art may better understand the aspects of the present disclosure. Those skilled in the art should appreciate that they may readily use the present disclosure as a basis for designing or modifying other processes and structures for carrying out the same purposes and/or achieving the same advantages of the embodiments introduced herein. Those skilled in the art should also realize that such equivalent constructions do not depart from the spirit and scope of the present disclosure, and that they may make various changes, substitutions, and alterations herein without departing from the spirit and scope of the present disclosure.

### EXAMPLES

While the present disclosure has been discussed in terms of certain embodiments, it should be appreciated that the present disclosure is not so limited. While certain embodiments were discussed in the present disclosure, it should be recognized that the present disclosure is not limited to only those embodiments. The embodiments are explained herein by way of example, and there are numerous modifications, variations and other embodiments that can be employed and would still be within the scope of the present disclosure.

### Example 1. Urea-Phospholipid Enhancement of Diclofenac Sodium Solubility

### Step 1: Preparation of urea and soya phosphatidylcholine (SPC) solution

In the aqueous solution containing 1% boric acid, add SPC (0%, 1%, 3%, 5%, 8%, 10%, 13%, 15%, 18%, 20%) and urea (0%, 5%, 10%, 20%, 30%, 40%, 50%) and stir until complete dissolution. A total of 70 sample solutions with different SPC and urea concentrations were obtained.

### Step 2: Diclofenac sodium solubility study

An excess of diclofenac sodium was added to each set of sample solutions and stirred overnight at 25°C. The sample solution was centrifuged at 12,000 rpm for 5 minutes, and a portion of the supernatant was diluted with appropriate diluent and analyzed by HPLC, to determine the solubility of diclofenac sodium in aqueous solutions containing different concentrations of SPC and urea. The results are presented in Table1.

Results in Table 1 indicated that the solubility of diclofenac sodium may be improved by urea or SPC when added separately or together. In the absence of SPC, when the concentration of urea increased from 0 to 30% urea, the solubility of diclofenac increased from 19.7 mg/mL to 68.3 mg/mL (3.5-fold) Urea can significantly improve the solubility of diclofenac. When the solution contains SPC, the solubility of diclofenac can be further improved. This shows that both SPC and urea have the effect of enhancing the solubility of diclofenac.

In the absence of urea, the solubility of diclofenac increased from 19.7 mg/mL to 96.8 mg/mL (4.9-fold) when SPC increased from 0% to 15%. After adding urea, the viscosity of the sample will decrease, so when an additional 5% urea is added, the SPC can be increased to 18%, the solubility of diclofenac can be increased to 116.5 mg/mL. When urea is added to 10%, SPC can be added to 20% , the solubility of diclofenac can be increased to 136.6 mg/mL. This shows that there is an interaction between urea and SPC to change the properties of the solution.

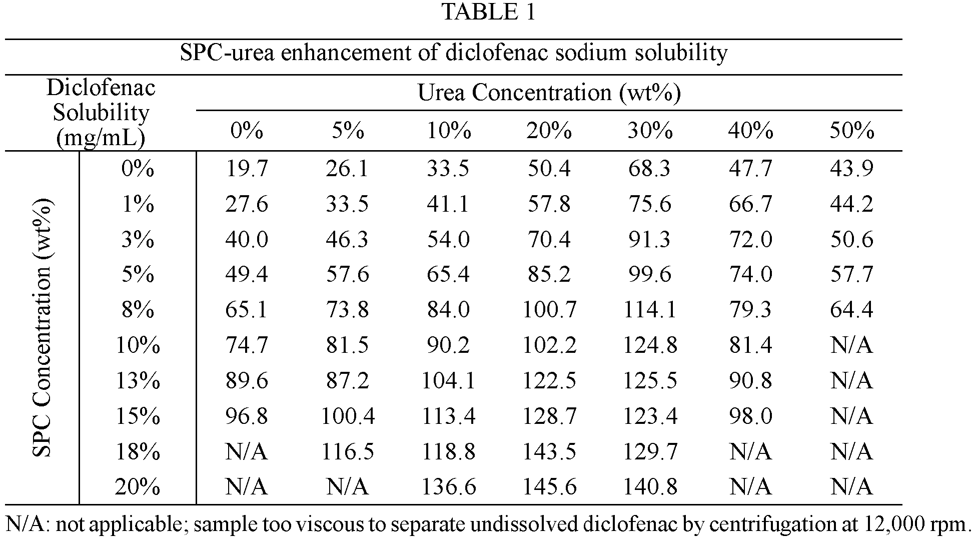

### Example 2. Variance in Amount of Diclofenac and SPC

### Step 1: Preparation of Oil Phase

A mixture was prepared by adding SPC and diclofenac in ethanol at R.T. until a homogeneous solution or suspension was obtained.

### Step 2: Preparation of Water Phase

Urea was added to any desired amount of water and stirred until urea was completely dissolved.

### Step 3: Mixing the Oil Phase and the Water Phase

The diclofenac solution was obtained by combining the Oil Phase and the Water Phase and mixing thoroughly until a homogeneous solution was obtained.

The formulations comprising diclofenac, SPC and urea are provided in TABLE 2.

As shown in TABLE 2, the concentrations of diclofenac in the formulations 1 to 8 varied from 2 wt% to 9 wt%. Stable formulations were also formed. A translucent solution was formed when the ratio of diclofenac sodium to SPC varied from 2:8 to 3:7. A transparent solution was formed when the ratio of diclofenac sodium to SPC varied from 4:6 to 8:2. As evidenced in formulation 12, precipitation occurred when the ratio of diclofenac sodium to SPC was increased to 9:1 because its solubility in the co-solvent vehicle was exceeded.

**TABLE 2**

| Exemplary formulations | | | | | | |
|---|---|---|---|---|---|---|
| # | Composition (wt%) | | | | | Performance |
| | Diclofenac | SPC | Urea | Ethanol | Water | Appearance |
| 1 | 2 | 8 | 30 | 10 | 50 | translucent solution |
| 2 | 3 | 7 | 30 | 10 | 50 | translucent gel |
| 3 | 4 | 6 | 30 | 10 | 50 | transparent solution |
| 4 | 5 | 5 | 30 | 10 | 50 | transparent solution |
| 5 | 6 | 4 | 30 | 10 | 50 | transparent solution |
| 6 | 7 | 3 | 30 | 10 | 50 | transparent solution |
| 7 | 8 | 2 | 30 | 10 | 50 | transparent solution |
| 8 | 9 | 1 | 30 | 10 | 50 | transparent solution with precipitate |

### Example 3. Stability of Diclofenac Solution

All of the formulations in TABLE 3 were prepared according to the processes described in Example 2.

The formulations comprising diclofenac are provided in TABLE 3.

As shown in TABLE 3, Formulation 11 without urea showed phase separation after stored at room temperature for 2 weeks. On the contrary, Formulation 9 and Formulation 10 of TABLE 3 with the ratio of diclofenac sodium to SPC between 4: 6 and 8: 2 was stable and remained stable after stored at room temperature for 2 weeks.

**TABLE 3**

| Exemplary formulations | | | | | | | | |
|---|---|---|---|---|---|---|---|---|
| # | Composition (wt%) | | | | | | Appearance | |
| | Diclofenac | SPC | Urea | Boric acid | Ethanol | Water | Initial | Stored at R.T. for 2 weeks |
| 9 | 4 | 5 | 10 | 1 | 10 | 70 | transparent solution | transparent solution |
| 10 | 4 | 10 | 10 | 1 | 10 | 65 | translucent solution | translucent solution |
| 11 | 4 | 10 | 0 | 1 | 10 | 75 | translucent solution | phase separation |

### Example 4. Variance in Type and Amount of First Generation and Second Generation NSAIDs

All of the formulations in TABLE 4 were prepared according to the processes described in Example 2.

Ibuprofen, ketoprofen, aceclofenac, naprofen, and indomethacin are classified as first generation NSAIDs, and piroxicam and meloxicam are classified as second generation NSAIDs which have COX2-selective inhibition activity. As seen in TABLE 4, when the ratio of first generation NSAIDs to SPC is between 2:5 to 5:3, stable formulations were also formed.

Ibuprofen, ketoprofen, and indomethacin have 5%, 2.5%, 1% and 0.5% of topical commercial product, respectively. These drugs also have higher solubility in this system and the formulations with actives of at least two-fold higher than commercial products were attained.

**TABLE 4**

| Exemplary formulations | | | | | | | |
|---|---|---|---|---|---|---|---|
| # | Composition (wt%) | | | | | | Performance |
| | NSAIDs type | SPC | Urea | pH adjuster | Ethanol | Water | Appearance |
| 12 | 10 (Ibuprofen) | 10 | 10 | 1 (Disodium phosphate) | 10 | 69 | transparent light-yellow solution |
| 13 | 4 (Ibuprofen) | 5 | 10 | 1 (Boric acid) | 10 | 70 | transparent solution |
| 14 | 5 (Ketoprofen) | 5 | 30 | 1 (Disodium phosphate) | 10 | 49 | translucent solution |
| 15 | 4 (Aceclofenac) | 5 | 10 | 1 (Boric acid) | 10 | 70 | transparent solution |
| 16 | 2 (Aceclofenac) | 5 | 10 | - | 10 | 73 | milky translucent solution |
| 17 | 5 (Naproxen) | 3 | 30 | 1 (Tris) | 10 | 51 | transparent sticky solution |
| 18 | 4 (Naproxen) | 5 | 10 | 1 (Boric acid) | 10 | 70 | translucent solution |
| 19 | 4 (Indomethacin) | 5 | 10 | 1 (Boric acid) | 10 | 70 | transparent solution |
| 20 | 2 (Piroxicam) | 3 | 30 | 1 (Sodium bicarbonate) | 10 | 54 | translucent solution |
| 21 | 4 (Piroxicam) | 5 | 10 | 1 (Boric acid) | 10 | 70 | creamy liquid |
| 22 | 2 (Meloxicam) | 3 | 30 | 1(Sodium bicarbonate) | 10 | 54 | milky translucent solution |

### Example 5. In Vitro Assay of Skin Permeation

An *in vitro* assay of skin permeation was performed with Franz diffusion cell for 48 hours at 32 ± 2°C, using porcine skin as a model membrane. Porcine skin was harvested from a female pig weighing 30-40 kg and further processed by dermatome (Zimmer, USA) so that the thickness was controlled to 0.45 ± 0.20 mm. The excised skin was carefully washed with phosphate buffered saline, dried with Kimwipes, and then preserved at -80 °C. Before use, the skin was thawed and then mounted on a Franz diffusion cell. The receptor compartment (3 mL) contains 20% ethanolic solution of degassed phosphate buffered saline, pH of 7.4, serving as a sink for the active. The *in vitro* assay was performed in a non-occlusive mode, and the effective diffusion area was 0.785 cm². 4 µL (160 ng diclofenac) of the Formulation 9 or the Formulation 23 or the Formulation 24 were applied to the donor compartment, and then spread uniformly over the skin by a small glass rod. The administration frequency of each sample was twice a day (BID). All of the Formulation 9, Formulation 23, and Formulation 24 in TABLE 5 were prepared according to the processes described in Example 2.

**TABLE 5**

| Exemplary formulations | | | | | | | |
|---|---|---|---|---|---|---|---|
| # | Composition (wt%) | | | | | | Performance |
| | Diclofenac | SPC | Urea | Boric acid | Ethanol | Water | Appearance |
| 9 | 4 | 5 | 10 | 1 | 10 | 70 | transparent solution |
| 23 | 4 | 5 | 0 | 1 | 10 | 80 | transparent solution |
| 24 | 4 | 0 | 10 | 1 | 10 | 75 | transparent solution |

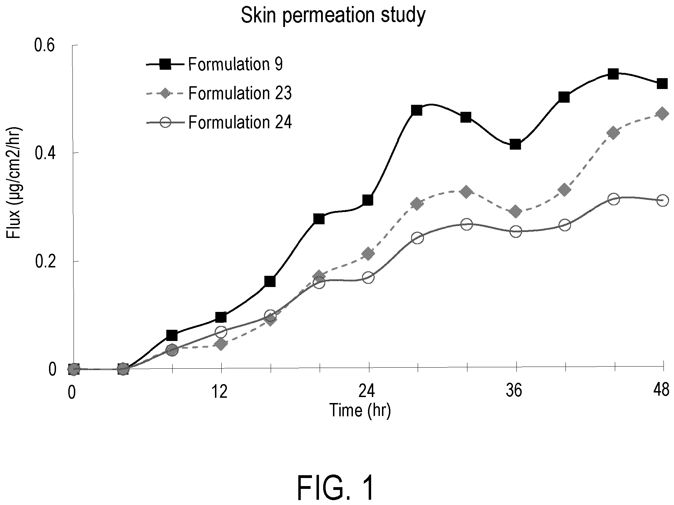

As observed in FIG. 1, the Formulation 9 comprising both of SPC and urea has better skin permeability. The diclofenac flux of Formulation 9 after dosing was higher than those of Formulation 23 (without urea) and Formulation 24 (without SPC). The combination of SPC and urea in the formulation promotes the penetration of diclofenac through the skin.

### Example 6. Effects of Dosing Regimen

Formulation 25 performed an *in vitro* assay of skin permeation according to the method of Example 5 and compared the effects of the following two dosing regimen on permeation profile. The first dosing regimen is a once-daily (QD) dose of 4 µL (160 ng diclofenac) per dose. The second dosing regimen is to halve the dosing volume (2 µL, 80 ng diclofenac), and the dosing frequency is twice-daily (BID), and the total daily dose is the same as the first method. The Formulation 25 in TABLE 6 was prepared according to the processes described in Example 2.

**TABLE 6**

| Exemplary formulation | | | | | | | | | | | |
|---|---|---|---|---|---|---|---|---|---|---|---|
| # | Composition (wt%) | | | | | | | | | | Performance |
| | Diclofenac | SPC | Urea | Boric acid | EDTA -2Na | BHT | PG | Ethanol | 10 N NaOH | Water | Appearance |
| 25 | 4 | 5 | 10 | 0.7 | 0.2 | 0.2 | 5 | 10 | 0.4 | 64.5 | transparent solution |

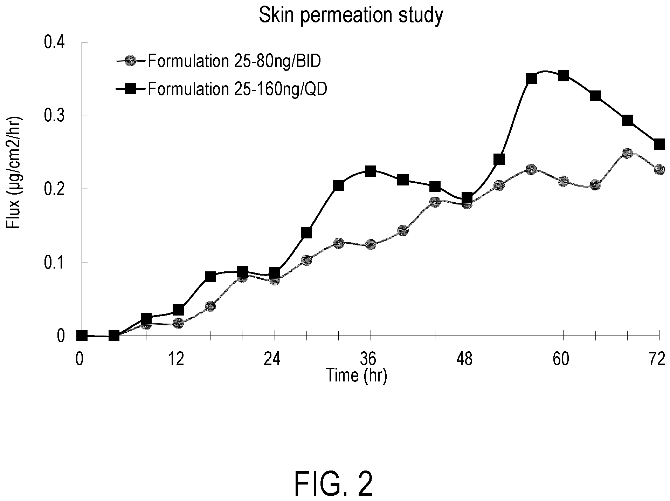

As observed in FIG.2, due to the higher dosing volume, the 160 ng/QD dosing regimen has better penetration rate than the one of 80 ng/BID dosing regimen in the first 12 hours. The 80 ng/BID dosing regimen was comparable to the 160 ng/QD dosing regimen in Flux at 24 hours after the second dose. However, by calculating the area under the curve (AUC), it was found that the AUC of the 160 ng/QD dosing regimen was higher than that of the 80 ng/BID, and the three consecutive days of dosing showed a similar trend.

### Example 7. Skin Irritation of the Compositions of the Present Disclosure

New Zealand White rabbits were used to evaluate all the formulations in Table 7. Prior to testing, the back of the rabbits was clipped free of hair. A volume of 32 µE (1.28 µg diclofenac) sample solution was applied directly to the skin, covering approximately an area of 2.5 × 2.5 cm². At the end of the 4-hour exposure time, the test samples were removed. After removing the test samples, all the sites were allowed to dry, and scored for erythema (score from 0 to 4) and edema (score from 0 to 4) at 24 ± 2 hours, 48 ± 2 hours and 72 ± 2 hours according to Draize scoring system. The Primary Irritation Index (PII) was calculated by scoring all animals for erythema and edema over three scoring times.

All of the formulations in TABLE 7 were prepared according to the processes described in Example 2.

**TABLE 7**

| Exemplary formulations | | | | | | | | | | | | | |
|---|---|---|---|---|---|---|---|---|---|---|---|---|---|
| # | Composition (wt%) | | | | | | | | | | Performance | | |
| | Diclofenac | SPC | Urea | Boric acid | BHT | EDTA-2Na | PG | Ethanol | 10 N NaOH | Water | Appearance | PII* | Response Category |
| 26 | 4 | 5 | 10 | 1 | 0 | 0.1 | 0 | 10 | 0.2 | 69.7 | transparent solution | 0.0 | Negligible |
| 27 | 0 | 5 | 10 | 1 | 0 | 0.1 | 0 | 10 | 0.2 | 73.7 | translucent solution | 0.0 | Negligible |
| 28 | 4 | 5 | 10 | 1 | 0 | 0.1 | 5 | 10 | 0.4 | 64.5 | transparent solution | 0.0 | Negligible |
| 29 | 4 | 3 | 20 | 0.7 | 0.2 | 0.2 | 5 | 10 | 0.3 | 56.6 | transparent solution | 0.0 | Negligible |

| | | | | | | | | | | | | | |
|---|---|---|---|---|---|---|---|---|---|---|---|---|---|
| *Primary Irritation Index | | | | | | | | | | | | | |

### Example 8. Comparison of Skin Penetration Effect with Commercial Product - 2% Pennsaid.

Formulation 30 performed an *in vitro* skin permeation study according to the method of Example 5. 4 µL of the Formulation 30 (160 ng diclofenac) or commercial product, i.e. 2% Pennsaid (80 ng diclofenac), were applied. The administration frequency of Formulation 30 was once-daily (QD), and the frequency of 2% Pennsaid was twice-daily (BID).

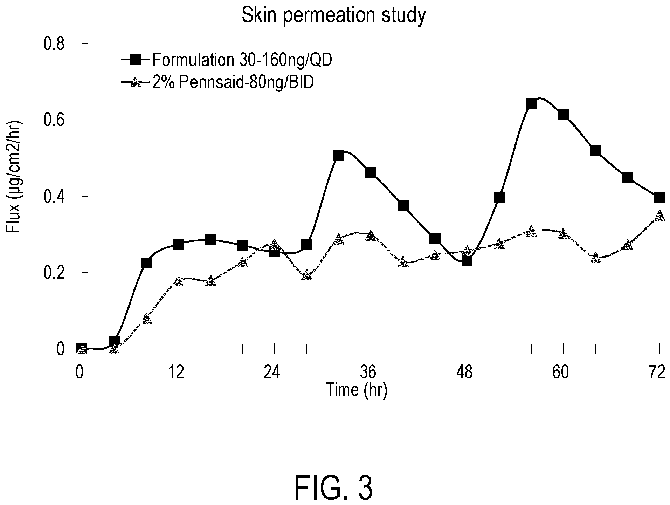

As observed in FIG.3, the 160 ng/QD dosing regimen of Formulation 30 have faster post-dose penetration, with Flux reaching Cₘₐₓ at 8 hours post-dose and decreasing to be comparable to 2% Pennsaid (80 ng diclofenac) with BID administration per the product label by 24 hours post-dose.

Formulation 30 at 160 ng/QD has a shorter Tₘₐₓ and higher AUC than 2% Pennsaid 80 ng/BID. This trend can be deduced that the diclofenac solution using urea-phospholipid system can have a shorter onset time and better efficacy than the commercial product.

**TABLE8**

| Exemplary formulation | | | | | | | | | |
|---|---|---|---|---|---|---|---|---|---|
| # | Composition (wt%) | | | | | | | | Performance |
| | Diclofenac | SPC | Urea | Boric acid | EDTA -2Na | PG | Ethanol | Water | Appearance |
| 30 | 4 | 5 | 10 | 1 | 0.1 | 5 | 10 | 64.9 | transparent solution |

## Claims

1. A topical composition comprising:
at least one active agent;
at least one phospholipid and urea; and
wherein the active agent comprises a non-steroid anti-inflammatory drug (NSAID).

2. The topical composition of claim 1, wherein the active agent is present at an amount of between about 0.1 wt% to about 10.0 wt% of the total composition.

3. The topical composition of claim 1 or 2, wherein the non-steroid anti-inflammatory drug (NSAID) is selected from the group consisting of acetylsalicylicacid, sodium salicylate, choline magnesium trisalicylate, salsalate, diflunisal, salicylsalicylic acid, sulfasalazine, olsalazin, acetaminophen, indomethacin, sulindac, etodolac, tolmetin, diclofenac, ketorolac, ibuprofen, naproxen, flurbiprofen, ketoprofen, aceclofenac, fenoprofen, oxaprozin, mefenamic, meclofenamic acid, piroxicam, meloxicam, tenoxicam, phenylbutazone, oxyphenbutazone, nabumetone, rofecoxib, celecoxib, any pharmaceutically acceptable salt, derivative and mixtures thereof.

4. The topical composition of any one of claims 1 to 3, wherein the phospholipid is present at an amount of between about 0.1 wt% to about 20.0 wt% of the total composition.

5. The topical composition of any one of claims 1 to 4, wherein the phospholipid is selected from the group consisting of phosphatidyl choline (PC), dipalmitoylphosphatidylcholine (DPPC), distearoyl phosphatidyl choline (DSPC), palmytoyl stearoyl phosphatidyl choline (PSPC), palmitoyl oleyl phosphatidyl choline (POPC), dioleyl phosphatidylcholine (DOPC), stearoyl oleyl phosphatidylcholine (SOPC), cardiolipin, plasmalogen, lyso phosphatidyl choline (LPC), phosphatidyl ethanolamine (PE) (Cephalin), phosphatidyl inositol (PI), phosphatidyl serine (PS), lecithin, lysolecithin, soy lecithin, rapeseed lecithin, corn or sunflower lecithins, egg lecithin, Epicorn 200, Epicorn 100, phospholipone 90G, LIPOID R-100 (Rapeseed), LIPOID H-100 (Sunflower), LIPOID-S100 (Soybean), LIPOID-S75, Phosal 50PG, and combinations thereof.

6. The topical composition of any one of claims 1 to 5, wherein the urea is present at an amount of between about 5.0 wt% to about 50.0 wt% of the total composition.

7. The topical composition of any one of claims 1 to 6 further comprises a lower chain alcohol having carbon chain length of C2 to C5.

8. The topical composition of claim 7, wherein the lower chain alcohol is selected from the group consisting of ethanol, propanol, isopropanol, butanol, isobutanol, pentanol, isopentanol, ethylene glycol, propylene glycol, butylene glycol, isobutylene glycol, pentylene glycol, isopentylene glycol and combinations thereof.

9. The topical composition of any one of claims 1 to 8 further comprises a pH adjusting agent.

10. The topical composition of claim 9, wherein the pH adjusting agent is selected from the group consisting of sodium phosphate, disodium phosphate, sodium bicarbonate, tris(hydroxymethyl)aminomethane, boric acid, sodium hydroxide, hydrochloride, triethylamine, citric acid, alanine, glycine, leucine, lactic acid, phenol and combinations thereof.

11. The topical composition of any one of claims 1 to 10, wherein the composition is in the form of cream, ointment, gel, transdermal formulations, foam, spray, lotion, solution, emulsion or suspension.

12. The topical composition of any one of claims 1 to 11, wherein the composition comprises about 0.1 wt% to about 10.0 wt% of active agent, about 0.1 wt% to about 20.0 wt% of phospholipid and about 5.0 wt% to about 50.0 wt% of urea, wherein the active agent comprises a non-steroid anti-inflammatory drug (NSAID).

13. Use of the topical composition of claim 1 for the manufacture of a medicament for treating inflammation, arthritis and/or pain, or conditions for which the signs and symptoms include inflammation, arthritis and/or pain.

14. The use of claim 13, wherein in the topical composition, the active agent is present at an amount of between about 0.1 wt% to about 10.0 wt% of the total composition.

15. The use of claim 13 or 14, wherein in the topical composition, the non-steroid anti-inflammatory drug (NSAID) is selected from the group consisting of acetylsalicylicacid, sodium salicylate, choline magnesium trisalicylate, salsalate, diflunisal, salicylsalicylic acid, sulfasalazine, olsalazin, acetaminophen, indomethacin, sulindac, etodolac, tolmetin, diclofenac, ketorolac, ibuprofen, naproxen, flurbiprofen, ketoprofen, fenoprofen, oxaprozin, mefenamic, meclofenamic acid, piroxicam, meloxicam, tenoxicam, phenylbutazone, oxyphenbutazone, nabumetone, rofecoxib, celecoxib, any pharmaceutically acceptable salt, derivative and mixtures thereof.

16. The use of claim 13, wherein in the topical composition, the phospholipid is present at an amount of between about 0.1 wt% to about 20.0 wt% of the total composition; or wherein the phospholipid is selected from the group consisting of phosphatidyl choline (PC), dipalmitoylphosphatidylcholine (DPPC), distearoyl phosphatidyl choline (DSPC), palmytoyl stearoyl phosphatidyl choline (PSPC), palmitoyl oleyl phosphatidyl choline (POPC), dioleyl phosphatidylcholine (DOPC), stearoyl oleyl phosphatidylcholine (SOPC), cardiolipin, plasmalogen, lyso phosphatidyl choline (LPC), phosphatidyl ethanolamine (PE) (Cephalin), phosphatidyl inositol (PI), phosphatidyl serine (PS), lecithin, lysolecithin, soy lecithin, rapeseed lecithin, corn or sunflower lecithins, egg lecithin, Epicorn 200, Epicorn 100, phospholipone 90G, LIPOID R-100 (Rapeseed), LIPOID H-100 (Sunflower), LIPOID-S100 (Soybean), LIPOID-S75, Phosal 50PG, and combinations thereof.

17. The use of any one of claims 13 to 16, wherein in the topical composition, the urea is present at an amount of between about 5.0 wt% to about 50.0 wt% of the total composition.

18. The use of claim 13, wherein the topical composition further comprises a lower chain alcohol having carbon chain length of C2 to C5.

19. The use of claim 18, wherein the lower chain alcohol is selected from the group consisting of ethanol, propanol, isopropanol, butanol, isobutanol, pentanol, isopentanol, ethylene glycol, propylene glycol, butylene glycol, isobutylene glycol, pentylene glycol, isopentylene glycol and combinations thereof; or wherein the topical composition further comprises a pH adjusting agent; or wherein the pH adjusting agent is selected from the group consisting of sodium phosphate, disodium phosphate, sodium bicarbonate, tris(hydroxymethyl)aminomethane, boric acid, sodium hydroxide, hydrochloride, triethylamine, citric acid, alanine, glycine, leucine, lactic acid, phenol and combinations thereof; or wherein the topical composition is in the form of cream, ointment, gel, transdermal formulations, foam, spray, lotion, solution, emulsion or suspension .

20. A method for preparation of a topical composition, the method comprising: (a) dissolving at least one active agent and at least one phospholipid to form an oil phase, (b) adding urea to water to form an aqueous phase, and (c) mixing the oil phase and the aqueous phase to form a homogenous composition, wherein the active agent comprises a non-steroid anti-inflammatory drug (NSAID).
